# EUROPEAN PATENT APPLICATION

(11) **EP 4 250 310 A1**
(43) Date of publication of application: **27.09.2023**
(21) Application number: 23163050.0
(22) Date of filing: 21.03.2023
(51) Int. Cl.: G16H 20/17, G16H 40/63

(54) **INFUSION DEVICE HUB FOR INTELLIGENT OPERATION OF INFUSION ACCESSORIES**

(30) Priority: 22.03.2022 US 202263322628 P
(71) Applicant: Carefusion 303 Inc., San Diego, California 92130 (US)
(72) Inventor: Mooney, Keith, San Diego, 92130 (US); O'Brien, Kevin, San Diego, 92130 (US)
(74) Representative: Zenz Patentanwälte Partnerschaft mbB

(57) **Abstract**

A device hub is communicatively connected to an infusion device and accessory devices associated with the infusion device. The infusion device monitors the hub for one or more status indicators associated with the accessory devices and obtains, via the hub, a plurality of status indictors, each indicating that a message is available from a respective accessory device associated with the status indicator without obtaining contents of the available message. The status indicators are prioritized based on a predetermined priority algorithm and a type of the respective accessory device, and a first accessory device is selected based on the prioritizing. The contents of the available message are obtained from the selected first accessory device without obtaining contents of other messages, and one or more parameters of the infusion device are adjusted based on the obtained contents before obtaining the contents of the other messages via the device hub.

## Description

### TECHNICAL FIELD

The present disclosure is generally related to a control device configured facilitate operation of accessories associated with an infusion device.

### BACKGROUND

Hospital infusion devices including pumps may include modular infusion platforms that are expandable with a various accessory devices to control and/or monitor infusions. Accessories typically communicate to infusion devices through analog or digital signal wires and require manual setup which can be time consuming. Different patients require different levels of treatment, and different accessories may be used with different patients and/or procedures, resulting in a wide variety of parameter variations that may conflict with each other during infusion and other medication delivery procedures. Failure to maintain control over the accessories may increase the possibility of human error, as well accessory damage, and increase the health risk to the patient.

### SUMMARY

According to various aspects, the subject technology provides a system and method for intelligently operating infusion accessory devices. In this regard, an intelligent accessory system is disclosed for use with infusion pumps and related devices. The system increases safety by reducing human error and accessory damage. The system is designed to work with smart accessories that contain a microcontroller and firmware, and which may automatically setup parameters on the pump thereby reducing the setup complexity for the user.

According to various aspects, a disclosed infusion system includes a device hub including at least two connection ports; and an infusion device in communication with the device hub, wherein the device hub is configured to: provide, to the infusion device, an indication that the device hub is communicatively connected to a plurality of accessory devices; and provide, to the infusion device, a plurality of status indictors, each status indicator indicating that a message is available from a respective accessory device attached to one connection port of the device hub that is associated with the status indicator without obtaining contents of the available message; and wherein the infusion device is configured to: receive the indication that the device hub is communicatively connected to the plurality of accessory devices; monitor the device hub for one or more status indicators associated with the plurality of accessory devices; obtain, from the device hub, the plurality of status indictors based on the monitoring; select, based on a priority for the one or more status indicators, a first accessory device of the plurality of accessory devices; obtain the contents of the available message from the selected first accessory device without obtaining contents of other messages; and adjust one or more parameters of the infusion pump based on the obtained contents before obtaining the contents of the other messages via the device hub.

According to various aspects, the infusion device is further configured to: obtain, from the device hub, an indication that a second accessory device was newly connected to the device hub; obtain a device type for the second accessory device; determine one or more setup parameters for the second accessory device based on the device type; and provide the one or more setup parameters to the second accessory device via the device hub. According to some aspects, the one or more setup parameters provided to the second accessory device comprise a parameter threshold, and the second accessory device is configured to: periodically compare, during operation, a measured parameter value with the parameter threshold, and only signal the infusion device via the device hub when the measured parameter value satisfies the parameter threshold. Other aspects include corresponding methods, apparatuses and computer program products for implementation of the corresponding system and its features.

A method includes receiving an indication that a device hub is communicatively connected to a plurality of accessory devices associated with an infusion device; periodically monitoring the device hub for one or more status indicators associated with the plurality of accessory devices; obtaining, via the device hub, a plurality of status indictors based on the monitoring, each status indicator indicating that a message is available from a respective accessory device associated with the status indicator without obtaining contents of the available message; prioritizing the plurality of status indicators based on a predetermined priority algorithm and a type of the respective accessory device associated with each of the plurality of status indicators; selecting, based on the prioritizing of the plurality of status indicators, a first accessory device of the plurality of accessory devices; obtaining the contents of the available message from the selected first accessory device without obtaining contents of other messages; and adjusting one or more parameters of the infusion device based on the obtained contents before obtaining the contents of the other messages via the device hub. Other aspects include corresponding systems, apparatus (e.g., an infusion device), and computer program products for implementation of the corresponding method and its features.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description of Implementations below, in conjunction with the following drawings. Like reference numerals refer to corresponding parts throughout the figures and description.
FIG. 1 depicts an example of an institutional patient care system of a healthcare organization, according to aspects of the subject technology.
FIG. 2A is a conceptual diagram illustrating an example system, including an example intelligent device hub, for intelligent operation of infusion accessory devices, according to aspects of the subject technology.
FIG. 2B depicts an example intelligent device hub configured to prioritize messages from connected accessories, according to aspects of the subject technology.
FIG. 3 depicts an example process for connecting an example accessory device to an infusion device via the disclosed intelligent device hub, according to aspects of the subject technology.
FIG. 4 depicts an example process for intelligent operation of infusion accessory devices, according to aspects of the subject technology.
FIG. 5 is a conceptual diagram illustrating an example electronic system 500 for intelligent operation of infusion accessory devices, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

The subject technology provides an Intelligent Infusion Device hub (IDH), including one or more microprocessors for communicating with various infusion accessories utilized during an infusion of a medication, including receiving and processing accessory related messages to and from the accessories. In various implementations, the IDH provides an interface between an IV infusion pump and one or more different accessory devices associated with the infusion pump, and provides parameters that are to be used for controlling infusions to a patient. According to various aspects disclosed herein, the IDH may incorporate control software (including, e.g., one or more algorithms) configured to prioritize accessory communications and facilitate configuration of the accessories for and during an administration of a medication to a patient.

The IDH may be part of the infusion pump or, in some implementations, be an external device separate from the pump and which communicates with the pump via a wired or wireless connection (e.g., USB and/or Bluetooth). In some implementations, the IDH is circuitry integrated within an infusion device. In some implementations, the IDH is an external controller configured to broker communications between the pump system and/or infusion device and the various connected accessory devices. The IDH may further be configured to add wireless, Bluetooth and LAN connection to pump systems that do not currently have it available. Adding such communications to the pump system may enable other capabilities such as remote control and or monitoring of the accessory devices. By having the electrical and processing components separate from the pump, the IDH facilitates integration of additional capabilities without needing to modify the pump's housing and electronics.

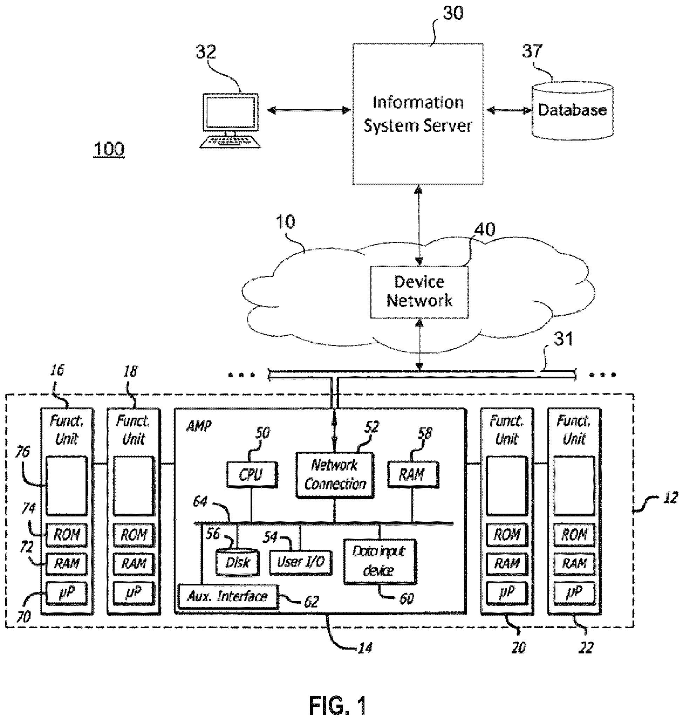

FIG. 1 depicts an example of an institutional patient care system 100 of a healthcare organization, according to aspects of the subject technology. In FIG. 1, a patient care device (or "medical device" generally) 12 is connected to a hospital network 10. The term patient care device (or "PCD") may be used interchangeably with the term patient care unit (or "PCU"), either which may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices. Each element 12 is connected to an internal healthcare network 10 by a transmission channel 31. Transmission channel 31 is any wired or wireless transmission channel, for example an 802.11 wireless local area network (LAN). In some implementations, network 10 also includes computer systems located in various departments throughout a hospital. For example, network 10 of FIG. 1 optionally includes computer systems associated with an admissions department, a billing department, a biomedical engineering department, a clinical laboratory, a central supply department, one or more unit station computers and/or a medical decision support system. As described further below, network 10 may include discrete subnetworks. In the depicted example, network 10 includes a device network 40 by which patient care devices 12 (and other devices) communicate in accordance with normal operations.

Additionally, institutional patient care system 100 may incorporate a separate information system server 30, the function of which will be described in more detail below. Moreover, although the information system server 30 is shown as a separate server, the functions and programming of the information system server 30 may be incorporated into another computer, if such is desired by engineers designing the institution's information system. Institutional patient care system 100 may further include one or multiple device terminals 32 for connecting and communicating with information system server 30. Device terminals 32 may include personal computers, personal data assistants, mobile devices such as laptops, tablet computers, augmented reality devices, or smartphones, configured with software for communications with information system server 30 via network 10.

Patient care device 12 comprises a system for providing patient care, such as that described in U.S. Pat. No. 5,713,856 to Eggers et al., which is incorporated herein by reference for that purpose. Patient care device 12 may include or incorporate pumps, physiological monitors (e.g., heart rate, blood pressure, ECG, EEG, pulse oximeter, and other patient monitors), therapy devices, and other drug delivery devices may be utilized according to the teachings set forth herein. In the depicted example, patient care device 12 comprises a interface device 14, also referred to as interface unit 14, connected to one or more functional modules 16, 18, 20, 22. Interface unit 14 includes a central processing unit (CPU) 50 connected to a memory, for example, random access memory (RAM) 58, and one or more interface devices such as user interface device 54, a coded data input device 60, a network connection 52, and an auxiliary interface 62 for communicating with additional modules or devices. Interface unit 14 also, although not necessarily, includes a main non-volatile storage unit 56, such as a hard disk drive or non-volatile flash memory, for storing software and data and one or more internal buses 64 for interconnecting the aforementioned elements.

In various implementations, user interface device 54 is a touch screen for displaying information to a user and allowing a user to input information by touching defined areas of the screen. Additionally, or in the alternative, user interface device 54 could include any means for displaying and inputting information, such as a monitor, a printer, a keyboard, softkeys, a mouse, a track ball and/or a light pen. Data input device 60 may be a bar code reader capable of scanning and interpreting data printed in bar coded format. Additionally, or in the alternative, data input device 60 can be any device for entering coded data into a computer, such as a device(s) for reading a magnetic strip, radio-frequency identification (RFID) devices whereby digital data encoded in RFID tags or smart labels (defined below) are captured by the reader 60 via radio waves, PCMCIA smart cards, radio frequency cards, memory sticks, CDs, DVDs, or any other analog or digital storage media. Other examples of data input device 60 include a voice activation or recognition device or a portable personal data assistant (PDA). Depending upon the types of interface devices used, user interface device 54 and data input device 60 may be the same device. Although data input device 60 is shown in FIG. 1 to be disposed within interface unit 14, it is recognized that data input device 60 may be integral within pharmacy system 34 or located externally and communicating with pharmacy system 34 through an RS-232 serial interface or any other appropriate communication means. Auxiliary interface 62 may be an RS-232 communications interface, however any other means for communicating with a peripheral device such as a printer, patient monitor, infusion pump or other medical device may be used without departing from the subject technology. Additionally, data input device 60 may be a separate functional module, such as modules 16, 18, 20 and 22, and configured to communicate with controller 14, or any other system on the network, using suitable programming and communication protocols.

Network connection 52 may be a wired or wireless connection, such as by Ethernet, WiFi, BLUETOOTH, an integrated services digital network (ISDN) connection, a digital subscriber line (DSL) modem, or a cable modem. Any direct or indirect network connection may be used, including, but not limited to a telephone modem, an MIB system, an RS232 interface, an auxiliary interface, an optical link, an infrared link, a radio frequency link, a microwave link, a personal area network connection, a local area network connection, a cellular link, or a WLANS connection or other wireless connection.

Functional modules 16, 18, 20, 22 are any devices for providing care to a patient or for monitoring patient condition. As shown in FIG. 1, at least one of functional modules 16, 18, 20, 22 may be an infusion pump module such as an intravenous infusion pump for delivering medication or other fluid to a patient. For the purposes of this discussion, functional module 16 is an infusion pump module. Each of functional modules 18, 20, 22 may be any patient treatment or monitoring device including, but not limited to, an infusion pump, a syringe pump, a patient controlled analgesia (PCA) pump, an epidural pump, an enteral pump, a blood pressure monitor, a pulse oximeter, an EKG monitor, an EEG monitor, a heart rate monitor, or an intracranial pressure monitor or the like. Functional module 18, 20 and/or 22 may be a printer, scanner, bar code reader or any other peripheral input, output or input/output device.

Each functional module 16, 18, 20, 22 communicates directly or indirectly with interface unit 14, with interface unit 14 providing overall monitoring and control of device 12. Functional modules 16, 18, 20, 22 may be connected physically and electronically in serial fashion to one or both ends of interface unit 14 as shown in FIG. 1, or as detailed in Eggers et al. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without connected through a separate interface unit or control unit 14. As described above, additional medical devices or peripheral devices may be connected to patient care device 12 through one or more auxiliary interfaces 62.

Each functional module 16, 18, 20, 22 may include module-specific components 76, a microprocessor 70, a volatile memory 72 and a nonvolatile memory 74 for storing information. It should be noted that while four functional modules are shown in FIG. 1, any number of devices may be connected directly or indirectly to central controller 14. The number and type of functional modules described herein are intended to be illustrative, and in no way limit the scope of the subject technology. Module-specific components 76 include any components necessary for operation of a particular module, such as a pumping mechanism for infusion pump module 16.

While each functional module may be capable of a least some level of independent operation, interface unit 14 monitors and controls overall operation of device 12. For example, as will be described in more detail below, interface unit 14 provides programming instructions to the functional modules 16, 18, 20, 22 and monitors the status of each module.

Patient care device 12 is capable of operating in several different modes, or personalities, with each personality defined by a configuration database. The configuration database may be a database 56 internal to patient care device, or an external database 37. A particular configuration database is selected based, at least in part, by patient-specific information such as patient location, age, physical characteristics, or medical characteristics. Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or a patient care device's 10 location in the hospital or hospital computer network. Patient care information may be entered through interface device 52, 54, 60 or 62, and may originate from anywhere in network 10, such as, for example, from a pharmacy server, admissions server, laboratory server, and the like.

Medical devices incorporating aspects of the subject technology may be equipped with a network interface module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 12 and network 10 may communicate via automated interaction, manual interaction, or a combination of both automated and manual interaction. Automated interaction may be continuous or intermittent and may occur through direct network connection 54 (as shown in FIG. 1), or through RS232 links, MIB systems, RF links such as BLUETOOTH, IR links, PANS, LANS, WLANS, digital cable systems, telephone modems or other wired or wireless communication means. Manual interaction between patient care device 12 and network 10 involves physically transferring, intermittently or periodically, data between systems using, for example, user interface device 54, coded data input device 60, bar codes, computer disks, portable data assistants, memory cards, or any other media for storing data. The communication means in various aspects is bidirectional with access to data from as many points of the distributed data sources as possible. Decision-making can occur at a variety of places within network 10. For example, and not by way of limitation, decisions can be made in HIS server 30, decision support 48, remote data server 49, hospital department or unit stations 46, or within patient care device 12 itself.

All direct communications with medical devices operating on a network in accordance with the subject technology may be performed through information system server 30, known as the remote data server (RDS). In accordance with aspects of the subject technology, network interface modules incorporated into medical devices such as, for example, infusion pumps or vital signs measurement devices, ignore all network traffic that does not originate from an authenticated RDS. The primary responsibilities of the RDS of the subject technology are to track the location and status of all networked medical devices that have NIMs, and maintain open communication

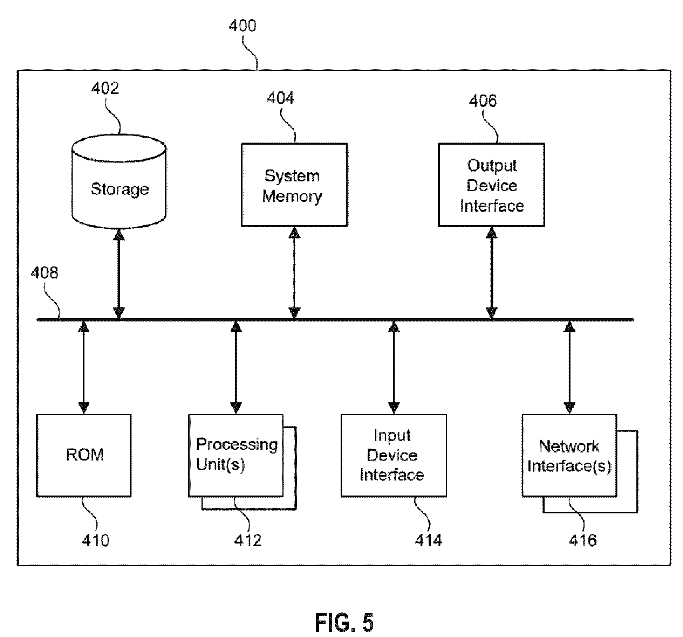

In some implementations, medication delivery modules 16, 18, 20, 22 include plug-in ports for expansion. Accordingly, a new medication delivery module may be attached to PCU 12 by coupling a connector through the plug-in ports, which may include electrical terminals so that the added medication delivery module 16, 18, 20, 22 may transmit and receive information to and from a control module 14. In some implementations, the added medication delivery module 16, 18, 20, 22 may also receive power from control module 14 through a plug-in port. Control module 14 may include a main display, a memory and a processor (see FIG. 5), and may be configured to display operational parameters and medication delivery status, and further information associated with each of medication delivery modules 16, 18, 20, 22. According to various implementations, module displays may also display physiological data (e.g., vital signs) associated with a patient.

A main display (e.g., I/O 54) may be configured to display one or more user interfaces for the display of operational parameters or other data associated with a module 16, 18, 20, 22, and/or physiological parameters associated with the patient. The main display may include multiple user interfaces, with each individual user interface graphically displaying information for a respective one of medication modules, including information also displayed on a corresponding module displays. In some implementations, control module 14 includes a communications module (including, e.g., an antenna), configured to communicate wirelessly with a controller, or with a network.

With reference to FIG. 1, when a medication delivery module 16, 18, 20, 22 initiates an infusion of a medication to the patient, the control module 14 is configured to create and manage an infusion session within a memory of the control module (or related module). For the purpose of this disclosure, the infusion session includes state information of the PCU 12, its control module 14, and/or its associated modules, which is recorded and saved to memory during a particular period of time. The state information includes, but is not limited to, records of parameter values utilized by the PCU, its control module, and/or its associated modules during the period of time, and/or records physiological data collected during the period of time. During the infusion, physiological data associated with the patient is recorded within the session, operating parameter values, and any modifications to the operating parameters of the PCU, its control module, and/or modules are also recorded in the session.

If not already logged into the PCU 12, the clinician may scan his or her badge proximate to a sensor (e.g., 54, 60) on the PCU 12, and the PCU may attempt to authenticate the clinician by sending the clinician's scanned identification to server 30. The clinician's badge may incorporate a radio frequency identification device (RFID), which is read by a scanner integrated with the PCU, or a portable scanner associated with the PCU. The clinician may scan his or her badge at the control module 14 to identify and authorize the clinician to initiate the administration of a medication. Once the clinician is associated with the PCU and/or module(s), the clinician's identification is associated with the session. The same is applicable with a patient. The clinician may scan the patient's wristband with a portable scanner, or using the sensor on the PCU 14 (or its control module) to associate the patient with the PCU and/or module(s) (and a session).

The control unit 14 of PCU 12 is configured to generate a graphical representation of the infusion session, and display (e.g., in a display) the graphical representation, including a graphical visualization of all parameters of the infusion during the session and any modifications any modifications to the parameters, together with physiological data obtained during the session. The graphical representation may include pseudo identifiers for unknown data until such data is substituted with known identifiers. At that time, the graphical representation is displayed with the known patient identifiers

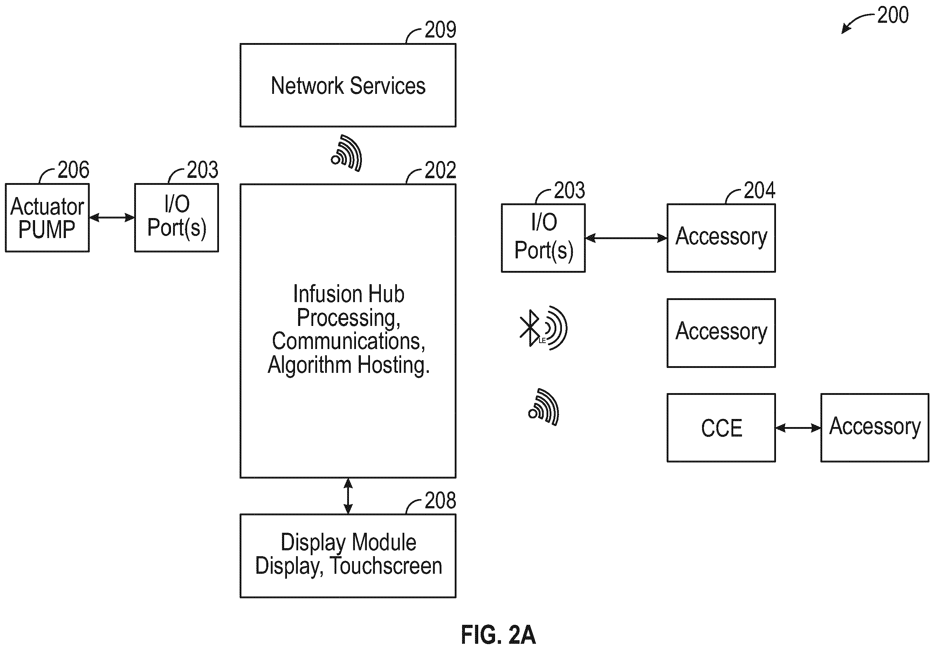

FIG. 2A is a conceptual diagram illustrating an example system, including an example intelligent device hub (IDH), for intelligent operation of infusion accessory devices, according to aspects of the subject technology. In the depicted example, an IDH 202 includes multiple configurable I/O ports 203 that provide bi-directional communication with accessory devices 204. The IDH may be simultaneously connected to one or more medical devices including, for example, one or more infusion pumps 206. In some implementations, the IDH may be implemented as or in a medication delivery module. In some implementations, the IDH may be implemented as or in

Each accessory device 204 may include a microcontroller that communicates with a microcontroller of the IDH 202 to facilitate a secure handshake with the IDH 202 and/or infusion device 206. The IDH 202, together with a corresponding infusion device and accessory devices, forms an intelligent accessory system that increases safety by reducing human error and accessory damage. In some implementations, the IDH 202 may include software instructions configured to facilitate automatic setup (e.g., on the infusion device) of infusion parameters related to a connected accessory. In some implementations, each accessory may include software or firmware configured to provide self-diagnosis of hardware/firmware issues which can then be communicated to the infusion device 206 via the IDH 202. The IDH 202 may further communicate with each connected accessory device 204 to ensure proper functionality (e.g., via reporting from the accessory) and automatically disable accessories which do not report a valid status, thereby preventing users from utilizing the wrong or damaged accessory.

According to various implementations, the IDH 202 includes multiple hardware ports 203, each including a universal connector. All connected accessories communicate via a common internal bus, alleviating the need for dedicated ports; that is, any accessory device 204 may be connected to any I/O port 203 to communicate with the infusion device 206 via a common digital communication protocol. In this regard, new accessories that implement a common protocol associated with the IDH 202 and which utilize the connector can be implemented without updates to the IDH 202 or the infusion device 206. Software updates may be pushed to the accessories via the IDH 202.

Accessories may require analog or digital I/O with specific power supply requirements, which may also be provided by ports 203. Additionally or in the alternative, I/O port 203 may include wireless connection points to suit the external accessory needs, e.g., analog, optical, custom wireless such as ultra-wideband (UWB), Zigbee, or Bluetooth low energy. The I/O port 203 may contain additional processing i.e., microcontroller, or digital signal processor hardware. A respective I/O port may be designed to support multiple sensors or support a single vendor supplied sensor.

According to various implementations, embedded firmware incorporated in the IDH 202 for each I/O port 203 and/or software drivers supporting the I/O port types may form an extensible "plug and play" capability to accommodate various device accessories and/or to connect to external IDHs. In some implementations, such as for external IDHs, I/O ports 203 may be added to the IDH 202 as needed. In some implementations, further communications with external systems may be accomplished over a network interface 205. In one example, a variety of sources may be connected to the IDH 202 to support control decisions by the infusion device.

IV infusion devices 12 or other delivery devices may directly communicate with the IDH 202 using serial, wired or wireless network connectivity (ethernet or WIFI), Other wireless connectivity such as BLE. Where specialized connectivity might be required then a predetermined I/O port may be loaded for the port(s) 203 corresponding to the type of device requiring such connectivity. Accordingly, external devices that may be connected to the IDH 202 may include, for example, a badge reader (e.g., for tasks such as RFID or NFC tap to associate, patients, sensors, pumps, clinician login), a biometric identification device (e.g., a fingerprint or retina scanner), or a backup battery for power loss or ambulatory usage.

The IDH 202 may also include networking hardware for wirelessly connecting with a wireless hub or other network device 209 of network 10, 40. In some implementations, the IDH 202 may be connected remotely to a PCU 12 or infusion pump through the server 30 and/or network 10, 40. The IDH 202 may facilitate network communications with server 30, PCU 12, or other network-enabled devices operably connected to a cloud-based system (e.g., via the network 10, 40). In this regard, patient information may be downloaded by the IDH 202 from an external system in the same or similar manner as data is received from connected accessory devices 204. For example, patient information may include limits of an infusion (e.g., volume, duration, infusion tubing pressure, quantity of air in line, etc.), or a flow rate of the infusion, provided by the infusion device.

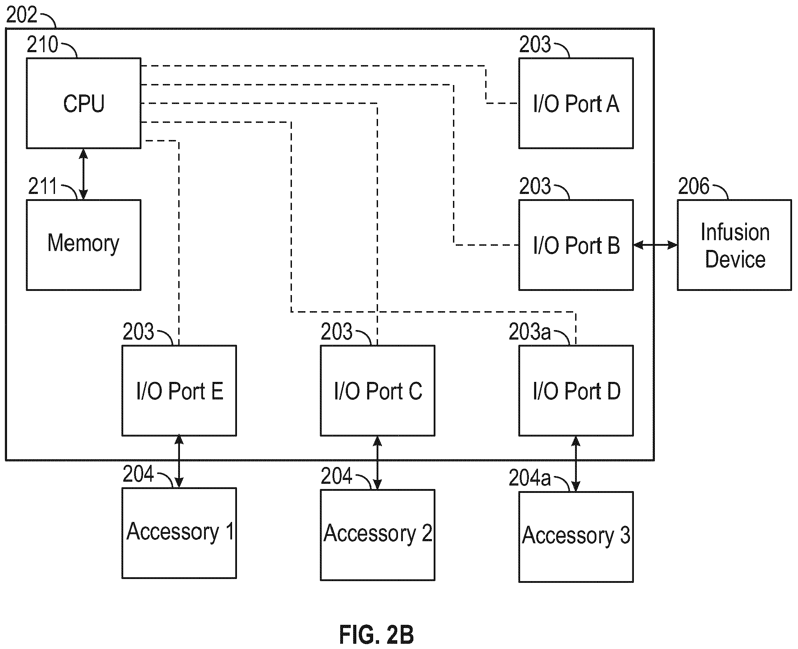

FIG. 2B depicts an example intelligent device hub configured to prioritize messages from connected accessories, according to aspects of the subject technology. In the depicted example, an intelligent device hub includes a plurality of I/O ports 203 and at least one processor 210. According to various implementations, the IDH 202 may include a predetermined number of hardware I/O ports 203 which each may be dynamically assigned an address corresponding to an internal I/O port.

When an accessory device 204a is connected to IDH 202, the processor 210 detects the newly connected accessory device 204a at the connected port 203a (e.g., by detecting a high or low signal). The processor 210 may assign an internal bus address to each newly connected device (e.g., as they are connected). The processor 210 may maintain internal bus addresses and assignments within a memory 211. When an infusion device 206 is connected and registered in memory 211, the processor 211 may alert the infusion device of the new connection between the device hub and a newly connected accessory device 204a. The processor 210 may then receive, from the infusion device 206 based on the alerting, an address that the infusion device 206 will use for the newly connected accessory device and a request for information pertaining to the new accessory device.

The processor 210 may authenticate the new accessory device. For example, the processor 210 may receive an authentication token from the new accessory device. The authentication token may be received from a memory of the new accessory device or be encoded in a readable component of the new accessory device. For example, the new accessory device may include a connector that can be received by the I/O port of the IDH. The connector may include a digitally (e.g., token, digital certificate, etc.), optically (e.g., barcode, quick read code, color code, light pattern, etc.), or physically (e.g., prongs, slots, slats, bevels, etc.) encoded value that can be detected by a corresponding accessory authorization sensor included in the IDH. The sensor of the IDH may be activated when the accessory is coupled to the I/O port. In such instances, the I/O port may include a switch or sensor to determine when an accessory is properly seated in the I/O port. Using the encoded value, the IDH may confirm via local or remote processing whether the new accessory device is authorized for use. If the accessory is not confirmed, the IDH may prevent connection between the new accessory device and the infusion device.

If authorized, the processor 210 may then facilitate a handshaking between the infusion device and the newly connected accessory device. The processor 210 may obtain the requested information pertaining to the new accessory device and provide the information to the infusion device. Such information may include operating parameters of the accessory device that the infusion device may require to process data received from the accessory device. According to various implementations, the information includes a type of the accessory device. In this regard, the infusion device may use the device type to query an outside system (e.g., server 30) for parameters to operate in accordance with the accessory device 204a. As another example, the accessory device may provide an error metric indicating the quality of a value provided by the accessory device. The error metric may be stored in the accessory device at manufacture time. In some implementations, the error metric may be dynamically generated by the accessory device based on, for example, self-testing. This allows the accessory device to provide an error metric that reflects the current state of the accessory device rather than a static value. In some implementations, the infusion device may independently confirm the authorization of the IDH and/or accessory using one or more of the techniques described with reference to the authorization of the accessory by the IDH.

The processor 210 is configured to receive, from the infusion device 206 (or server 30), one or more accessory parameters for operating the accessory device independent of the infusion device, and to provide the one or more accessory parameters to the newly connected accessory device. As will be described further, the accessory device 204a is configured to operate independently of the infusion device based on the one or more accessory parameters and to report status via the connected port 203a. The parameters may include, for example, operating limits within which the accessory device should operate and/or at which the accessory device should warn the infusion pump when met or exceeded. The accessory device 204a may signal the IDH 202 when it is configured to operate (independently of the infusion device) based on the one or more accessory parameters.

IDH 202 is configured to transmit, to the infusion device 206, an indication when it is communicatively connected to a plurality of accessory devices (e.g., via ports 203). When a respective accessory device 204 is ready to transmit a status to the infusion device 206, the IDH 202 broadcasts a status indicator for the device 204. When multiple accessory devices are ready to transmit device status then the IDH 202 may broadcast status indicators for all of the devices 204. According to some implementations, broadcasting includes providing the status indicator(s) responsive to a query from the infusion device 206.

According to various implementations, a priority is assigned to each connected accessory device 204 based on the type of device (e.g., received in the information) and, in some implementations, the type of other connected devices. The priority may be assigned further based on a treatment profile currently programmed into the infusion device 206. The priority may be assigned and/or reassigned as each device is connected. According to various implementations, the priority is assigned and managed by the connected infusion device 206. In some implementations, the priority may be assigned and/or reassigned by the IDH 202. A prioritization scheme may be stored locally by the infusion device, for example, in memory 56 or 58, or stored remotely in a remote database 37 and downloaded as needed. In some implementations, the prioritization scheme may be stored in memory 211 of the IDH 202. When more than one connected device 204 is ready to transmit a status, the processor 210 may provide status indicators for all of the devices to the infusion device, and the infusion device may decide which device's 204 message to receive first based on the prioritization scheme.

Some accessories may be predetermined to have a higher priority than others. Accordingly, a message waiting on one channel (e.g., bolus, patient controlled/demand) may be ignored based on a message waiting on a higher priority message (e.g. occlusion alert). In some implementations, sensor devices 204 which result in stopping infusions may be set to the highest priority. A message associated with a pressure sensor may take priority over a flow sensor because an occlusion causing the increased pressure may be what is causing a low flow condition detected by the flow sensor. Similarly, certain lower priority devices may be ignored, or even not activated by the infusion device, until higher priority messages have been processed. For example, barcode scanning and setup may be ignored or deactivated while an alert condition of a higher priority device is active, or until another higher priority action has been completed (e.g., the infusion set being fully primed). An example prioritization scheme is shown in Table 1, below.

**Table 1 - Prioritization Scheme for Accessory Device Types**

| **Accessory** | **Accessory specific events** | **Neonatal enteral nutrition priority profile** | **Neonatal priority profile** | **Oncology priority profile** | **Life sustaining priority profile** | **Pain management priority profile** |
|---|---|---|---|---|---|---|
| Pressure sensor | Upstream occlusion | 7 | 6 | 6 | 6 | 10 |
| | Downstream occlusion | 3 | 2 | 2 | 5 | 2 |
| Air detection | Air in line | 8 | 1 | 1 | 1 | 1 |
| Drop sensor | No flow-Low flow | 5 | NA | 4 | 3 | 6 |
| | Flow error-Unexpected drops detected | 6 | NA | 5 | 9 | 7 |
| | High flow-Free flow | 2 | NA | 3 | 8 | 9 |
| Inline flow sensor | High flow | 1 | 4 | NA | 7 | 8 |
| | Low flow | 4 | 5 | NA | 2 | 5 |
| Bolus button | Bolus request | NA | NA | NA | NA | 4 |
| SpO2 sensor | Low oxygen level | NA | 3 | NA | 4 | 3 |
| Barcode scanner | Information scanned | NA | NA | NA | NA | NA |

Where two devices may be responsible for the same alert (e.g., occlusion and air-in-line), the infusion device may instruct one or more accessories to stand down the alert until the alert from the higher priority device is processed; at which point the infusion device may instruct (e.g., via the IDH 202) to perform a recheck to confirm that the alert condition is still present.

Accessory devices 204 may include smart accessories that contain a microcontroller and firmware. A smart accessory may perform a handshake procedure with the infusion pump, automatically setup parameters on the pump which reduce the setup complexity for the use. In this regard, a smart accessory device 204 may be configured to alert the host (e.g., the IDH 202 and/or the infusion device 206) that something has happened, at which point the host can, with a single transaction, determine which accessory raised the alert, and then query the nature of the alert from the respective accessory. In some implementations, an alarm system may be implemented on the host, to be triggered if an alert is set, but not responded to by the host in within a predetermined time.

In some implementations, for a sensor-based accessory device (i.e. a drop sensor), one or more thresholds may be sent to the accessory as part of the initialization/configuration process, allowing for monitoring independent of the host. For user-input type accessories, the host may not need to poll the device regularly, instead only responding to an alert. In some implementations, accessory devices 204 may include built-in self-testing, where on initialization, key parameters may be checked to ensure proper operation, and any errors communicated to the host (e.g., via IDH 202). Key parameters may also be monitored during operation to detect any failures with the accessory.

Computer program code for carrying out operations of the subject technology may be written in an object-oriented programming language such as, for example, JAVA^{®}, Smalltalk, or C++. However, the computer program code for carrying out operations of the subject technology may also be written in conventional procedural programming languages, such as the "C" programming language, in an interpreted scripting language, such as Perl, or in a functional (or fourth generation) programming language such as Lisp, SML, Forth, or the like. The software may also be written to be compatible with HLA-7 requirements.

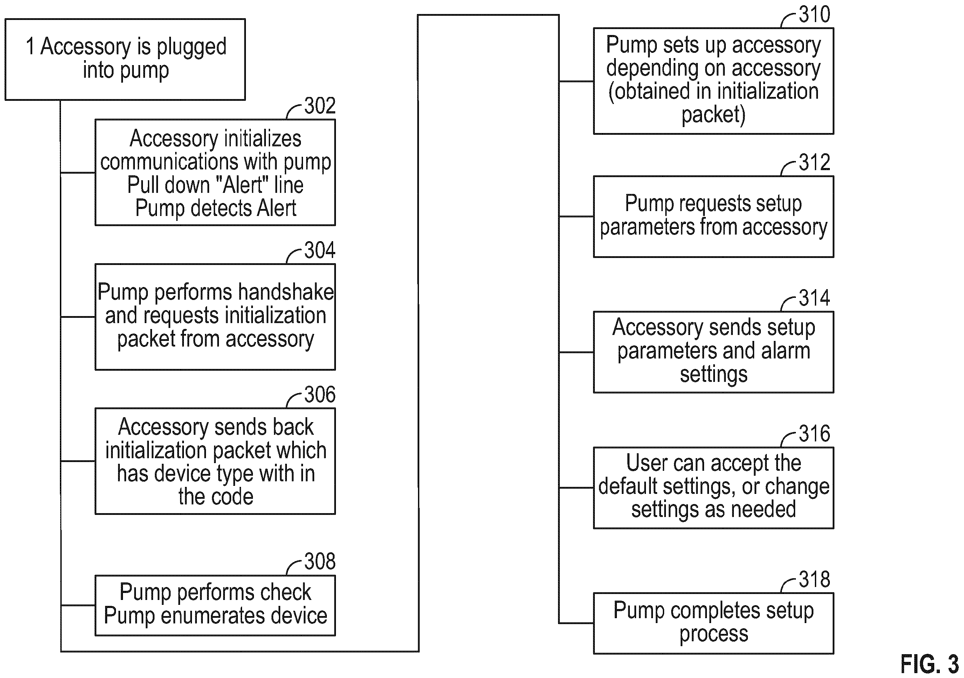

FIG. 3 depicts an example process for connecting an example accessory device to an infusion device via the disclosed intelligent device hub, according to aspects of the subject technology. For explanatory purposes, the various blocks of example process 300 are described herein with reference to FIGS. 1 and 2, and the components and/or processes described herein. The one or more of the blocks of process 300 may be implemented, for example, by one or more computing devices including, for example, IDH 202, or component thereof. In some implementations, one or more of the blocks may be implemented apart from other blocks, and by one or more different processors or devices. Further, for explanatory purposes, the blocks of example process 300 are described as occurring in serial, or linearly. However, multiple blocks of example process 300 may occur in parallel. In addition, the blocks of example process 300 need not be performed in the order shown and/or one or more of the blocks of example process 300 need not be performed.

According to the depicted example, when an accessory device (e.g., a drop sensor) is connected to the infusion pump, the accessory device initializes communication with the pump (302), for example, by signaling on a channel of the bus (e.g., by pulling down an alert line). The pump detects the signal and performs a handshake, assigns an address to the device (e.g., 0x00 for the first accessory), and requests an initialization packet from the accessory device (304). The accessory sends back an initialization packet which includes its device type with the code assigned to the device (e.g., 0x00) (306). The pump then performs predetermined checks for the accessory type and assigns an address (e.g., 0x00) to the accessory device (308). The pump sets up the accessory (410), according to the accessory type (e.g., obtained in the initialization packet).

The pump then requests setup parameters from the accessory (312). The accessory then sends the setup parameters and alarm settings to the pump (314). The pump may then present the settings (e.g., on a display device) to the user for confirmation (316). In some implementations, the pump may present the clinician with limits determined for the accessory (e.g., on the display device). The user may then accept the default settings, or change settings as needed (416). The pump completes the setup process (318).

In an example wherein a drop sensor is used, the pump may calculate drops per time period based on flow settings inputted by the user, calculate limits, and provide the drop sensor accessory device with the limits so that the accessory knows when to contact the pump (e.g., when the limits are exceeded). For example, the pump may provide the accessory with a threshold at which the accessory should alarm the pump. In this regard, the pump no longer has to deal with the accessory until the accessory itself determines an action is needed and provides a message to the pump during operation. The pump may then decide whether to take an action based on the message and a predetermined prioritization scheme. In some implementations, the message provided by the accessory device may include multiple threshold alert levels (e.g., hard or soft limit exceeded) and provide the current alert level with the message. The pump may assign different priorities to different thresholds and prioritize the accessory message depending on the value provided with the message in respect to other messages and the priorities given to them.

Traditionally, a sensor continuously reports its status (including, e.g., measured value) and the pump continuously calculates whether the status has met certain conditions (e.g., is within the given threshold ranges or limits). Using the subject technology, the pump provides the accessory device with conditions that the pump determines are relevant and the accessory device reports back to the pump (using the bus) when those conditions are met. At that point, the pump may or may not act on the reporting based on the prioritization scheme and other messages that may also be on the bus.

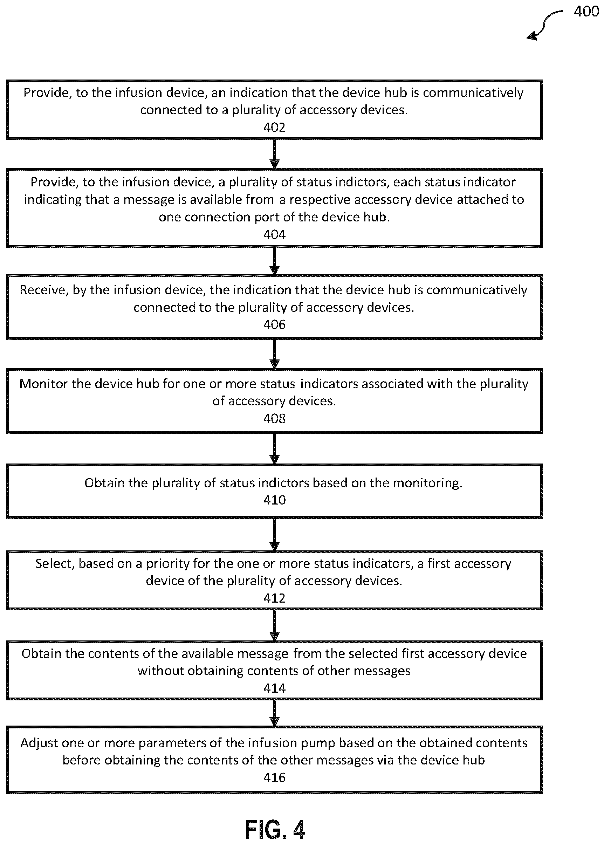

FIG. 4 depicts an example process for intelligent operation of infusion accessory devices, according to aspects of the subject technology. For explanatory purposes, the various blocks of example process 400 are described herein with reference to FIGS. 1 through 3, and the components and/or processes described herein. The one or more of the blocks of process 400 may be implemented, for example, by one or more computing devices including, for example, IDH 202, or component thereof. In some implementations, one or more of the blocks may be implemented apart from other blocks, and by one or more different processors or devices. Further for explanatory purposes, the blocks of example process 400 are described as occurring in serial, or linearly. However, multiple blocks of example process 400 may occur in parallel. In addition, the blocks of example process 400 need not be performed in the order shown and/or one or more of the blocks of example process 400 need not be performed.

The system executing the example process 400 includes an IDH 202 (or "device hub") including at least two connection ports 203, at least one accessory device in communication with the IDH via one of the ports, and an infusion device 206 in communication with the IDH via one of the ports. As described previously, the IDH 202 is configured to operably connect to the infusion device 206, for example, by way of a wireless pairing or by way of a wired connection.

In the depicted example, the IDH 202 is connected to a plurality of accessory devices. The IDH provides, to a connected infusion device 206, an indication that the device hub is communicatively connected to a plurality of accessory devices (402).

As described previously, when an accessory device is connected to the IDH 202, the IDH 202 may alert the infusion device of a new connection between the IDH 202 and the newly connected accessory device 204. The IDH 202 may then receive, from the infusion device 206 based on the alerting, an address for the newly connected accessory device along with a request for information pertaining to the new accessory device. The information may be obtained by the IDH 202 and provided to the infusion device, and the IDH 202 may receive (e.g., from the infusion device) one or more accessory parameters for operating the accessory device independent of the infusion device. The one or more accessory parameters may then be provided to the newly connected accessory device. In some implementations, the infusion device 206 and/or the IDH 202 may confirm that the newly connected accessory device is configured to operate independently of the infusion device based on the one or more accessory parameters and to report status via the device hub.

The IDH 202 provides, to the infusion device 206, a plurality of status indictors, each status indicator indicating that a message is available from a respective accessory device 204 attached to one connection port 203 of the IDH 202 that is associated with the status indicator (404). In this regard, each status indicator may be obtained without obtaining contents of the available message.

The infusion device 206 receives the indication that the IDH 202 is communicatively connected to the plurality of accessory devices (406), and begins to monitor the device hub for one or more status indicators associated with the plurality of accessory devices (408).

The infusion device 206 obtains, from the IDH 202, the plurality of status indictors based on the monitoring (410) and selects, based on a priority for the one or more status indicators, a first accessory device of the plurality of accessory devices (412).

Selecting the first accessory device based on the priority may include determining a treatment profile for an ongoing medication infusion by the infusion device. In this regard, the infusion device may provide a plurality of treatment profiles for selection at a user interface associated with the infusion device, and receive a user selection of the treatment profile from the plurality of treatment profiles. When evaluating status indicators for first and second accessory devices, the infusion device may then determine, based on the determined treatment profile, that the first accessory device has a higher priority than a second accessory device.

According to various implementations, certain types of accessory devices may be predetermined to have a higher priority than other types. For example, an automated infusion monitoring device such as a flow meter or pressure sensor may have a higher priority than a patient or clinician controlled accessory device such as a bolus button or barcode scanner. The patient controlled accessory may register with the IDH 202 a first status indicator, and the automated infusion monitoring device may register a second status indicator. In such an example, the infusion device 206 may obtain, based on detecting the second status indicator, a message associated with the second status indicator from the automated infusion monitoring device without obtaining a message associated with the first status indicator, and adjust the infusion based on the obtained message associated with the second status indicator. The first status indicator and the message associated with the first status indicator may be ignored until the infusion is adjusted based on the obtained message associated with the second status indicator.

When two or more status indicators are broadcast by the IDH 202, a prioritization scheme may be indexed based on a type of a first accessory device and a type of a second accessory device and a treatment profile associated with the infusion device. In some implementations, the prioritization scheme may be a table stored in memory (see Table 1). In some implementations, the IDH 202 indexes the table and selects between the first and second accessory devices based on a result of the indexing. In some implementations, the infusion device indexes the scheme and selects based on the result. The status message associated with the accessory device selected based on indexing the prioritization scheme may then be provided to or retrieved by the infusion device.

In some implementations, an alarm type may be provided with a status message (e.g., for at least one of the first and second accessory devices), and the indexing of the prioritization scheme may further include the alarm type, as shown in Table 1.

In some implementations, IDH 202 may include an accessory authentication sensor (e.g., an authorization accessory 204a). In this regard, the IDH 202 may be configured to, upon coupling with a respective accessory 204, activate the accessory authentication sensor, obtain an authentication value for the respective accessory 204 via the accessory authentication sensor, and determine, based at least in part on the authentication value, whether the accessory is unauthorized (or authorized). Messaging between the between the infusion device and the accessory may then be prevented responsive to determining that the accessory is unauthorized (or allowed responsive to determining that the accessory is authorized).

Continuing with process 400, the infusion device 206 then obtains the contents of the available message from the selected first accessory device without obtaining contents of other messages (414). That is, based on the selected status indictor, the infusion device may signal the IDH to retrieve and/or provide the corresponding message from the corresponding accessory device to the infusion device. In one example, if the selected status indicator is at a first address 0x01 then the infusion device may query the IDH based on the first address to obtain the message associated with the accessory device also associated with the first address. In some implementations, messages corresponding to each status indicator may be stored in memory 211 according to their addresses. Based on the obtained message, the one or more parameters of the infusion pump are adjusted (e.g., by the infusion device) before the contents of the other messages are obtained via the device hub (416).

The example process 400 may then optionally continue by the infusion device 206 obtaining, from the device hub, an indication that a second accessory device was newly connected to the device hub, obtaining a device type for the second accessory device, determining one or more setup parameters for the second accessory device based on the device type, and providing the one or more setup parameters to the second accessory device via the device hub. The one or more setup parameters provided to the second accessory device may include, for example, a parameter threshold. Once configured, the second accessory device may periodically compare, during operation, a measured parameter value with the parameter threshold, and only signal the infusion device via the device hub when the measured parameter value satisfies the parameter threshold.

Many of the above-described example 400, and related features and applications, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

FIG. 5 is a conceptual diagram illustrating an example electronic system 500 for intelligent operation of infusion accessory devices, according to aspects of the subject technology. Electronic system 500 may be a computing device for execution of software associated with one or more portions or steps of process 500, or components and processes provided by FIGS. 1-4, including but not limited to information system server 30, database 37, computing hardware within patient care device 12, or a remote device 32 (e.g., a mobile device). Electronic system 500 may be representative, in combination with the disclosure regarding FIGS. 1-4. In this regard, electronic system 500 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 500 may include various types of computer readable media and interfaces for various other types of computer readable media. In the depicted example, electronic system 500 includes a bus 508, processing unit(s) 512, a system memory 504, a read-only memory (ROM) 510, a permanent storage device 502, an input device interface 514, an output device interface 506, and one or more network interfaces 516. In some implementations, electronic system 500 may include or be integrated with other computing devices or circuitry for operation of the various components and processes previously described.

Bus 508 collectively represents all system, peripheral, and chipset buses that communicatively connect the numerous internal devices of electronic system 500. For instance, bus 508 communicatively connects processing unit(s) 512 with ROM 510, system memory 504, and permanent storage device 502.

From these various memory units, processing unit(s) 512 retrieves instructions to execute and data to process, in order to execute the processes of the subject disclosure. The processing unit(s) can be a single processor or a multi-core processor in different implementations.

ROM 510 stores static data and instructions that are needed by processing unit(s) 512 and other modules of the electronic system. Permanent storage device 502, on the other hand, is a read-and-write memory device. This device is a non-volatile memory unit that stores instructions and data even when electronic system 500 is off. Some implementations of the subject disclosure use a mass-storage device (such as a magnetic or optical disk and its corresponding disk drive) as permanent storage device 502.

Other implementations use a removable storage device (such as a floppy disk, flash drive, and its corresponding disk drive) as permanent storage device 502. Like permanent storage device 502, system memory 504 is a read-and-write memory device. However, unlike storage device 502, system memory 504 is a volatile read-and-write memory, such as a random access memory. System memory 504 stores some of the instructions and data that the processor needs at runtime. In some implementations, the processes of the subject disclosure are stored in system memory 504, permanent storage device 502, and/or ROM 510. From these various memory units, processing unit(s) 512 retrieves instructions to execute and data to process in order to execute the processes of some implementations.

Bus 508 also connects to input and output device interfaces 514 and 506. Input device interface 514 enables the user to communicate information and select commands to the electronic system. Input devices used with input device interface 514 include, e.g., alphanumeric keyboards and pointing devices (also called "cursor control devices"). Output device interfaces 506 enables, e.g., the display of images generated by the electronic system 500. Output devices used with output device interface 506 include, e.g., printers and display devices, such as cathode ray tubes (CRT) or liquid crystal displays (LCD). Some implementations include devices such as a touchscreen that functions as both input and output devices.

Also, as shown in FIG. 5, bus 508 also couples electronic system 500 to a network (not shown) through network interfaces 516. Network interfaces 516 may include, e.g., a wireless access point (e.g., Bluetooth or WiFi) or radio circuitry for connecting to a wireless access point. Network interfaces 516 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 500 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

### Illustration of Subject Technology as Clauses:

Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identification

Clause 1. An infusion system comprising: a device hub including at least two connection ports; and an infusion device in communication with the device hub, wherein the device hub is configured to: provide, to the infusion device, an indication that the device hub is communicatively connected to one or more accessory devices; and provide, to the infusion device, a plurality of status indictors, each status indicator indicating that a message is available from a respective accessory device attached to one connection port of the device hub that is associated with the status indicator without obtaining contents of the available message; and wherein the infusion device is configured to: receive the indication that the device hub is communicatively connected to the one or more accessory devices; monitor the device hub for one or more status indicators associated with the one or more accessory devices; obtain, from the device hub, the plurality of status indictors based on the monitoring; select, based on a priority for the one or more status indicators, a first accessory device of the one or more accessory devices; obtain the contents of the available message from the selected first accessory device without obtaining contents of other messages; and adjust one or more parameters of the infusion device based on the obtained contents before obtaining the contents of the other messages via the device hub.

Clause 2. The infusion system of Clause 1, wherein the infusion device is further configured to: obtain, from the device hub, an indication that a second accessory device was newly connected to the device hub; obtain a device type for the second accessory device; determine one or more setup parameters for the second accessory device based on the device type; and -provide the one or more setup parameters to the second accessory device via the device hub.

Clause 3. The infusion system of Clause 2, wherein the one or more setup parameters provided to the second accessory device comprise a parameter threshold, and wherein the second accessory device is configured to: periodically compare, during operation, a measured parameter value with the parameter threshold, and only signal the infusion device via the device hub when the measured parameter value satisfies the parameter threshold.

Clause 4. The infusion system of any one of Clauses 1 through 3 wherein selecting the first accessory device based on the priority comprises: determining a treatment profile for an ongoing medication infusion by the infusion device; and determining, based on the determined treatment profile, that the first accessory device has a higher priority than a second accessory device of the one or more accessory devices, the one or more status indicators including status indictors for the first and second accessory devices, and wherein the infusion device is further configured to, before selecting the first accessory device based on the priority: provide a plurality of treatment profiles for selection at a user interface associated with the infusion device; and receive a user selection of the treatment profile from the plurality of treatment profiles.

Clause 5. The infusion system of any one of Clauses 1 through 4, wherein the device hub includes an accessory authentication sensor, wherein the device hub is configured to: upon coupling with an accessory, activate the accessory authentication sensor; obtain an authentication value from the accessory via the accessory authentication sensor; determine, based at least in part on the authentication value, that the accessory is unauthorized; and prevent messaging between the infusion device and the accessory.

Clause 6. The infusion system of any one of Clauses 1 through 5, wherein the infusion device is further configured to: identify, from the device hub, a first status indicator associated with a patient controlled accessory and a second status indicator associated with an automated infusion monitoring device; obtain, based on identifying the second status indicator, a message associated with the second status indicator from the automated infusion monitoring device without obtaining a message associated with the first status indicator.

Clause 7. The infusion system of Claim 6, wherein the infusion device is further configured to: adjust the infusion based on the obtained message associated with the second status indicator; and ignore the first status indicator and the message associated with the first status indicator until the infusion is adjusted based on the obtained message associated with the second status indicator.

Clause 8. The infusion system of any one of Clauses 1 through 7, wherein the device hub is further configured to: alert the infusion device of a new connection between the device hub and a newly connected accessory device; receive, from the infusion device based on the alerting, an address for the newly connected accessory device and a request for information pertaining to the new accessory device; obtain and provide the information pertaining to the new accessory device to the infusion device; receive, from the infusion device, one or more accessory parameters for operating the accessory device independent of the infusion device; provide the one or more accessory parameters to the newly connected accessory device; and confirm that the newly connected accessory device is configured to operate independently of the infusion device based on the one or more accessory parameters and to report status via the device hub.

Clause 9. The infusion system of any one of Clauses 1 through 8, wherein the device hub or infusion device is further configured to: identify a first status indicator associated with a first accessory device and a second status indictor associated with a second accessory device; index a prioritization scheme based on a type of the first accessory device and a type of the second accessory device and a treatment profile associated with the infusion device; select between the first and second accessory devices based on a result of the indexing; and provide a status message associated with the selected accessory device to the infusion device.

Clause 10. The infusion system of Clause 9, wherein receiving the first and second status indicators comprises receiving an alarm type for at least one of the first and second accessory devices, and wherein the indexing further comprises indexing the prioritization scheme by the alarm type.

Clause 11. A machine-implemented method, comprising: receiving an indication that a device hub is communicatively connected to one or more accessory devices associated with an infusion device; periodically monitoring the device hub for one or more status indicators associated with the one or more accessory devices; obtaining, via the device hub, a plurality of status indictors based on the monitoring, each status indicator indicating that a message is available from a respective accessory device associated with the status indicator without obtaining contents of the available message; prioritizing the plurality of status indicators based on a predetermined priority algorithm and a type of the respective accessory device associated with each of the plurality of status indicators; selecting, based on the prioritizing of the plurality of status indicators, a first accessory device of the one or more accessory devices; obtaining the contents of the available message from the selected first accessory device without obtaining contents of other messages; adjusting one or more parameters of the infusion device based on the obtained contents before obtaining the contents of the other messages via the device hub.

Clause 12. The machine-implemented method of Clause 11, further comprising: obtaining, from the device hub, an indication that a second accessory device was newly connected to the device hub; obtaining a device type for the second accessory device; determining one or more setup parameters for the second accessory device based on the device type; and providing the one or more setup parameters to the second accessory device via the device hub.

Clause 13. The machine-implemented method of Clause 12, wherein the one or more setup parameters provided to the second accessory device comprise a parameter threshold, the method further comprising: periodically comparing, during operation, a measured parameter value with the parameter threshold, and only signal the infusion device via the device hub when the measured parameter value satisfies the parameter threshold.

Clause 14. The machine-implemented method of any one of Clauses 11 through 13, wherein selecting the first accessory device based on the prioritizing comprises: determining a treatment profile for an ongoing medication infusion by the infusion device; and determining, based on the determined treatment profile, that the first accessory device has a higher priority than a second accessory device of the one or more accessory devices, the one or more status indicators including status indictors for the first and second accessory devices, and wherein the infusion device is further configured to, before selecting the first accessory device based on the priority: providing a plurality of treatment profiles for selection at a user interface associated with the infusion device; and receiving a user selection of the treatment profile from the plurality of treatment profiles.

Clause 15. The machine-implemented method of Clause 14, wherein the device hub includes an accessory authentication sensor, wherein the device hub is configured to: upon coupling with an accessory, activating the accessory authentication sensor; obtaining an authentication value from the accessory via the accessory authentication sensor; determining, based at least in part on the authentication value, that the accessory is unauthorized; and preventing messaging between the infusion device and the accessory.

Clause 16. The machine-implemented method of any one of Clauses 11 through 14, further comprising: identifying, from the device hub, a first status indicator associated with a patient controlled accessory and a second status indicator associated with an automated infusion monitoring device; and obtaining, based on identifying the second status indicator, a message associated with the second status indicator from the automated infusion monitoring device without obtaining a message associated with the first status indicator.

Clause 17. The machine-implemented method of Clause 16, further comprising: adjusting the infusion based on the obtained message associated with the second status indicator; and ignoring the first status indicator and the message associated with the first status indicator until the infusion is adjusted based on the obtained message associated with the second status indicator.

Clause 18. The machine-implemented method of any one of Clauses 11 through 17, further comprising: detecting a new connection between the device hub and a newly connected accessory device; assigning, from the infusion device based on the detecting, an address for the newly connected accessory device and a request for information pertaining to the new accessory device; obtaining the information pertaining to the new accessory device; providing, to the new accessory device, one or more accessory parameters for operating the accessory device independent of the infusion device; and confirming that the newly connected accessory device is configured to operate independently of the infusion device based on the one or more accessory parameters and to report status via the device hub.

Clause 19. The machine-implemented method of any one of Clauses 11 through 18, further comprising: identifying a first status indicator associated with a first accessory device and a second status indictor associated with a second accessory device; indexing a prioritization scheme based on a type of the first accessory device and a type of the second accessory device and a treatment profile associated with the infusion device; selecting between the first and second accessory devices based on a result of the indexing; and providing a status message associated with the selected accessory device to the infusion device.

Clause 20. The machine-implemented method of Clause 19, wherein receiving the first and second status indicators comprises receiving an alarm type for at least one of the first and second accessory devices, and wherein the indexing further comprises indexing the prioritization scheme by the alarm type.

Clause 21. A non-transitory machine-readable medium storing instructions thereon that, when executed by a device, cause the device to perform a machine-implemented method according to any one of Clauses 11 through 20.

Clause 22. The non-transitory machine-readable medium of Clause 21, wherein the device is an infusion device or a device hub operably connected to an infusion device.

Clause 23. An infusion device configured to perform a method according to any one of Clauses 11 through 20.

### Further Consideration:

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to", "operable to", and "programmed to" do not imply any particular tangible or intangible modification of a subject, but, rather, are intended to be used interchangeably. For example, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some embodiments, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. An infusion system comprising:
a device hub including at least two connection ports; and
an infusion device in communication with the device hub,
wherein the device hub is configured to:
provide, to the infusion device, an indication that the device hub is communicatively connected to one or more accessory devices; and
provide, to the infusion device, a plurality of status indictors, each status indicator indicating that a message is available from a respective accessory device attached to one connection port of the device hub that is associated with the status indicator without obtaining contents of the available message; and
wherein the infusion device is configured to:
receive the indication that the device hub is communicatively connected to the one or more accessory devices;
monitor the device hub for one or more status indicators associated with the one or more accessory devices;
obtain, from the device hub, the plurality of status indictors based on the monitoring;
select, based on a priority for the one or more status indicators, a first accessory device of the one or more accessory devices;
obtain the contents of the available message from the selected first accessory device without obtaining contents of other messages; and
adjust one or more parameters of the infusion device based on the obtained contents before obtaining the contents of the other messages via the device hub.

2. The infusion system of Claim 1, wherein the infusion device is further configured to:
obtain, from the device hub, an indication that a second accessory device was newly connected to the device hub;
obtain a device type for the second accessory device;
determine one or more setup parameters for the second accessory device based on the device type; and
provide the one or more setup parameters to the second accessory device via the device hub.

3. The infusion system of Claim 2, wherein the one or more setup parameters provided to the second accessory device comprise a parameter threshold, and wherein the second accessory device is configured to:
periodically compare, during operation, a measured parameter value with the parameter threshold, and only signal the infusion device via the device hub when the measured parameter value satisfies the parameter threshold.

4. The infusion system of any one of Claims 1 through 3, wherein selecting the first accessory device based on the priority comprises:
determining a treatment profile for an ongoing medication infusion by the infusion device; and
determining, based on the determined treatment profile, that the first accessory device has a higher priority than a second accessory device of the one or more accessory devices, the one or more status indicators including status indictors for the first and second accessory devices, and
wherein the infusion device is further configured to, before selecting the first accessory device based on the priority:
provide a plurality of treatment profiles for selection at a user interface associated with the infusion device; and
receive a user selection of the treatment profile from the plurality of treatment profiles.

5. The infusion system of any one of Claims 1 through 4, wherein the device hub includes an accessory authentication sensor, wherein the device hub is configured to:
upon coupling with an accessory, activate the accessory authentication sensor;
obtain an authentication value from the accessory via the accessory authentication sensor;
determine, based at least in part on the authentication value, that the accessory is unauthorized; and
prevent messaging between the infusion device and the accessory.

6. The infusion system of any one of Claims 1 through 5, wherein the infusion device is further configured to:
identify, from the device hub, a first status indicator associated with a patient controlled accessory and a second status indicator associated with an automated infusion monitoring device;
obtain, based on identifying the second status indicator, a message associated with the second status indicator from the automated infusion monitoring device without obtaining a message associated with the first status indicator.

7. The infusion system of Claim 6, wherein the infusion device is further configured to:
adjust the infusion based on the obtained message associated with the second status indicator; and
ignore the first status indicator and the message associated with the first status indicator until the infusion is adjusted based on the obtained message associated with the second status indicator.

8. The infusion system of any one of Claims 1 through 7, wherein the device hub is further configured to:
alert the infusion device of a new connection between the device hub and a newly connected accessory device;
receive, from the infusion device based on the alerting, an address for the newly connected accessory device and a request for information pertaining to the new accessory device;
obtain and provide the information pertaining to the new accessory device to the infusion device;
receive, from the infusion device, one or more accessory parameters for operating the accessory device independent of the infusion device;
provide the one or more accessory parameters to the newly connected accessory device; and
confirm that the newly connected accessory device is configured to operate independently of the infusion device based on the one or more accessory parameters and to report status via the device hub.

9. The infusion system of any one of Claims 1 through 8, wherein the device hub or infusion device is further configured to:
identify a first status indicator associated with a first accessory device and a second status indictor associated with a second accessory device;
index a prioritization scheme based on a type of the first accessory device and a type of the second accessory device and a treatment profile associated with the infusion device;
select between the first and second accessory devices based on a result of the indexing; and
provide a status message associated with the selected accessory device to the infusion device.

10. The infusion system of Claim 9, wherein receiving the first and second status indicators comprises receiving an alarm type for at least one of the first and second accessory devices, and wherein the indexing further comprises indexing the prioritization scheme by the alarm type.

11. A machine-implemented method, comprising:
receiving an indication that a device hub is communicatively connected to one or more accessory devices associated with an infusion device;
periodically monitoring the device hub for one or more status indicators associated with the one or more accessory devices;
obtaining, via the device hub, a plurality of status indictors based on the monitoring, each status indicator indicating that a message is available from a respective accessory device associated with the status indicator without obtaining contents of the available message;
prioritizing the plurality of status indicators based on a predetermined priority algorithm and a type of the respective accessory device associated with each of the plurality of status indicators;
selecting, based on the prioritizing of the plurality of status indicators, a first accessory device of the one or more accessory devices;
obtaining the contents of the available message from the selected first accessory device without obtaining contents of other messages; and
adjusting one or more parameters of the infusion device based on the obtained contents before obtaining the contents of the other messages via the device hub.

12. The machine-implemented method of Claim 11, further comprising:
obtaining, from the device hub, an indication that a second accessory device was newly connected to the device hub;
obtaining a device type for the second accessory device;
determining one or more setup parameters for the second accessory device based on the device type; and
providing the one or more setup parameters to the second accessory device via the device hub.

13. The machine-implemented method of Claim 12, wherein the one or more setup parameters provided to the second accessory device comprise a parameter threshold, the method further comprising:
periodically comparing, during operation, a measured parameter value with the parameter threshold, and only signal the infusion device via the device hub when the measured parameter value satisfies the parameter threshold.

14. The machine-implemented method of any one of Claims 11 through 13, wherein selecting the first accessory device based on the prioritizing comprises:
determining a treatment profile for an ongoing medication infusion by the infusion device; and
determining, based on the determined treatment profile, that the first accessory device has a higher priority than a second accessory device of the one or more accessory devices, the one or more status indicators including status indictors for the first and second accessory devices, and
wherein the infusion device is further configured to, before selecting the first accessory device based on the priority:
providing a plurality of treatment profiles for selection at a user interface associated with the infusion device; and
receiving a user selection of the treatment profile from the plurality of treatment profiles.

15. The machine-implemented method of Claim 14, wherein the device hub includes an accessory authentication sensor, wherein the device hub is configured to:
upon coupling with an accessory, activating the accessory authentication sensor;
obtaining an authentication value from the accessory via the accessory authentication sensor;
determining, based at least in part on the authentication value, that the accessory is unauthorized; and
preventing messaging between the infusion device and the accessory.

16. The machine-implemented method of any one of Claims 11 through 15, further comprising:
identifying, from the device hub, a first status indicator associated with a patient controlled accessory and a second status indicator associated with an automated infusion monitoring device; and
obtaining, based on identifying the second status indicator, a message associated with the second status indicator from the automated infusion monitoring device without obtaining a message associated with the first status indicator.

17. The machine-implemented method of Claim 16, further comprising:
adjusting the infusion based on the obtained message associated with the second status indicator; and
ignoring the first status indicator and the message associated with the first status indicator until the infusion is adjusted based on the obtained message associated with the second status indicator.

18. The machine-implemented method of any one of Claims 11 through 17, further comprising:
detecting a new connection between the device hub and a newly connected accessory device;
assigning, from the infusion device based on the detecting, an address for the newly connected accessory device and a request for information pertaining to the new accessory device;
obtaining the information pertaining to the new accessory device;
providing, to the new accessory device, one or more accessory parameters for operating the accessory device independent of the infusion device; and
confirming that the newly connected accessory device is configured to operate independently of the infusion device based on the one or more accessory parameters and to report status via the device hub.

19. The machine-implemented method of any one of Claims 11 through 18, further comprising:
identifying a first status indicator associated with a first accessory device and a second status indictor associated with a second accessory device;
indexing a prioritization scheme based on a type of the first accessory device and a type of the second accessory device and a treatment profile associated with the infusion device;
selecting between the first and second accessory devices based on a result of the indexing; and
providing a status message associated with the selected accessory device to the infusion device.

20. The machine-implemented method of Claim 19, wherein receiving the first and second status indicators comprises receiving an alarm type for at least one of the first and second accessory devices, and wherein the indexing further comprises indexing the prioritization scheme by the alarm type.

21. A non-transitory machine-readable medium storing instructions thereon that, when executed by a device, cause the device to perform a machine-implemented method according to any one of Claims 11 through 20.

22. The non-transitory machine-readable medium of Claim 21, wherein the device is an infusion device or a device hub operably connected to an infusion device.

23. An infusion device configured to perform a method according to any one of Claims 11 through 20.
